(19) Europäisches Patentamt
European Patent Office
Office européen des brevets

(11) **EP 3 352 821 B1**

(12) **FASCICULE DE BREVET EUROPEEN**

(45) Date de publication et mention
de la délivrance du brevet:
**31.07.2019 Bulletin 2019/31**

(21) Numéro de dépôt: **16784225.1**

(22) Date de dépôt: **20.09.2016**

(51) Int Cl.:
**A61M 5/32** *(2006.01)*

(86) Numéro de dépôt international:
**PCT/FR2016/052377**

(87) Numéro de publication internationale:
**WO 2017/051108 (30.03.2017 Gazette 2017/13)**

(54) **ENSEMBLE DE DISTRIBUTION COMPORTANT UNE SERINGUE ET UN DISPOSITIF DE PROTECTION POUR AIGUILLE**

AUSGABEANORDNUNG MIT EINER SPRITZE UND EINEM NADELSCHUTZ

DISPENSING ASSEMBLY HAVING A SYRINGE AND A NEEDLE GUARD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.09.2015 FR 1558966**

(43) Date de publication de la demande:
**01.08.2018 Bulletin 2018/31**

(73) Titulaire: **Aptar Stelmi SAS**
**93420 Villepinte (FR)**

(72) Inventeurs:
• **FOURNIER, Ghislain**
**17000 La Rochelle (FR)**
• **SWAL, Mickaël**
**77124 Chauconin Neufmontiers (FR)**

(74) Mandataire: **CAPRI**
**33, rue de Naples**
**75008 Paris (FR)**

(56) Documents cités:
**EP-A1- 1 208 861    EP-A1- 1 964 588**

## Description

La présente invention est définie par les caractéristiques de la revendication 1 et concerne un ensemble de distribution comportant une seringue et un dispositif de protection pour aiguille.

Des dispositifs de protection pour aiguille de seringue, également appelés protèges-aiguilles, sont connus, notamment des documents EP0429052, EP0976415, EP1208861 et WO2015052417.

Toutefois, ce type de dispositif de protection pour aiguille peut présenter des inconvénients.

Les protèges-aiguilles actuels sont ainsi susceptibles de générer une goutte au niveau de la pointe de l'aiguille lors du retrait du protège-aiguille. Ce phénomène peut être gênant notamment en ce qui concerne la perte de produit, l'intégrité de l'ensemble, et l'inconvénient de devoir essuyer l'aiguille avant piqure ce qui engendre un essuyage du silicone présent sur l'aiguille, qui peut avoir pour conséquence une plus forte douleur lors de l'injection. Plus précisément, les protèges-aiguilles actuels, lors de leur retrait, génèrent une dépression à l'intérieur de la cavité interne. Cette dépression a pour conséquence une aspiration/succion au niveau de l'orifice de l'aiguille qui de ce fait aspire le liquide contenu dans la seringue. Cette aspiration, même minime, se traduit par l'apparition d'une goutte sur le biseau formant la pointe de l'aiguille. Ce phénomène peut être plus ou moins amplifié par la façon de retirer manuellement le protège-aiguille (en biais, lentement, en pinçant, etc.). Cela a pour conséquence un essuyage de l'aiguille, et potentiellement une dégradation de celle-ci. De plus, en cas d'utilisation dans un autoinjecteur, un protocole de nettoyage de la goutte ne peut pas être appliqué.

La présente invention a pour objectif de fournir un ensemble de distribution ne présentant pas les inconvénients précités.

Plus particulièrement, la présente invention a pour but de fournir un dispositif de protection pour aiguille de seringue qui garantit l'absence de formation de goutte à la sortie de l'aiguille au moment du retrait du dispositif de protection.

La présente invention a aussi pour but de fournir un dispositif de protection pour aiguille de seringue qui soit simple et peu coûteux à fabriquer et à assembler, et fiable d'utilisation.

La présente invention a donc pour objet un ensemble de distribution comportant une seringue comprenant un corps pourvu d'une boule supportant une aiguille pourvue d'une pointe, et un dispositif de protection amovible pour aiguille de seringue, ledit dispositif de protection comportant un capuchon élastique qui définit une cavité intérieure délimitée par une paroi latérale et par une paroi d'extrémité distale, ladite paroi d'extrémité étant pleine et recevant de manière étanche, en position de protection, ladite pointe de ladite aiguille d'une seringue, ladite paroi latérale comportant un bourrelet annulaire pourvu d'au moins une fente s'étendant en direction longitudinale sur ledit bourrelet annulaire, ledit bourrelet annulaire coopérant de manière étanche, en position de protection, avec ladite boule (104) de la seringue, ladite cavité dudit capuchon étant dimensionnée de telle sorte que lorsque ledit dispositif de protection est retiré de ladite seringue, ladite cavité est reliée à l'atmosphère via ladite au moins une fente avant que ladite pointe de l'aiguille soit reliée à ladite cavité.

Avantageusement, une pluralité, notamment quatre, fentes régulièrement angulairement réparties s'étendent en direction longitudinale sur ledit bourrelet annulaire.

Avantageusement, ladite au moins une fente a une longueur inférieure à la hauteur de ladite boule.

Avantageusement, ledit capuchon est réalisé conformément à l'équation suivante:

$$X < Y + Z/2$$

avec X étant la distance entre le point de fermeture étanche de la pointe de l'aiguille et le point d'étanchéité entre le capuchon et la boule, Y étant la longueur de l'aiguille entre la pointe et la surface d'extrémité de la boule, et Z étant la hauteur de la boule.

Avantageusement, ledit ensemble comporteen outre une coque rigide destinée à entourer en le contenant ledit capuchon et étant pourvue de moyens de retenue dudit capuchon.

Ces caractéristiques et avantages et d'autres de la présente invention apparaîtront plus clairement au cours de la description détaillée suivante, faite en référence aux dessins joints, donnés à titre d'exemples non-limitatifs, et sur lesquels :

les figures 1 à 4 sont des vues schématiques en section transversale d'un dispositif de protection d'aiguille selon un mode de réalisation avantageux de la présente invention, illustrant quatre étapes successives lors du retrait dudit dispositif de protection d'aiguille;

la figure 5 est une vue schématique en section transversale, illustrant plus en détail le dispositif de protection d'aiguille dans la position de la figure 4; et

la figure 6 est une vue partielle agrandie en section transversale, illustrant plus en détail le dispositif de protection d'aiguille dans la position de la figure 3.

Dans la description qui suit de la présente invention, les termes "proximal" et "distal" se réfèrent à l'arrière du corps de seringue, c'est-à-dire à la partie du corps de seringue qui est opposé à l'aiguille.

Sur les figures, il est représenté un dispositif de protection 10 pour aiguille de seringue, ou protège-aiguille. Ce dispositif de protection 10 est destiné à être assemblé de manière amovible sur une seringue 100.

Une telle seringue 100 comporte un corps de seringue 102 contenant un piston (non représenté), une partie proximale 103 ouverte et une partie distale 104 qui supporte l'aiguille A pourvue d'une pointe P définissant l'ouverture distale de l'aiguille A. Cette partie distale 104 est aussi appelée boule de la seringue, et c'est ce terme qui sera utilisé ci-après.

Le protège-aiguille 10 comporte un capuchon élastique 20 qui définit une cavité intérieure 26 délimitée par une paroi latérale 28 et par une paroi d'extrémité distale 30. La paroi d'extrémité 30 est pleine et a une épaisseur qui permet le logement de la pointe P de l'aiguille A. Le capuchon 20 est réalisé en matériau souple, typiquement du caoutchouc.

Avantageusement, le capuchon élastique 20 est de révolution autour de son axe central longitudinal. Cette symétrie de révolution concerne le contour externe du capuchon 20 (paroi latérale 28 et paroi d'extrémité 30) ainsi que le contour interne de la paroi latérale 28 qui définit la cavité 26, sauf en ce qui concerne la présence de fentes et de rainures comme il sera expliqué ci-après.

Dans l'exemple de réalisation représenté sur les figures, le dispositif de protection 10 pour aiguille de seringue comporte, outre le capuchon élastique 20, une coque rigide 80 dans laquelle est logé le capuchon 20. La présence de cette coque rigide 80 n'est toutefois pas obligatoire pour la présente invention.

Ce type de coque 80 est classiquement utilisé pour renforcer la protection de l'utilisateur de la seringue contre une piqûre par aiguille en offrant une protection supplémentaire extérieure rigide qui est difficilement percée par l'aiguille 106. Cette coque rigide 80 présente une forme générale longitudinale cylindrique de section circulaire, elle est montée de manière coaxiale par rapport au capuchon 20. La coque rigide 80 est dimensionnée pour permettre l'insertion et le blocage du capuchon 20 à l'intérieur. A cet effet, la coque rigide 80 présente une forme intérieure qui suit sensiblement la forme extérieure du capuchon 20. Afin de retenir le capuchon 20 à l'intérieur de la cavité 86 de la coque rigide 80, sont prévus des moyens de retenue du capuchon comprenant un rebord rentrant 96, de préférence annulaire, qui forme un élément, de préférence une collerette, faisant saillie vers l'intérieur.

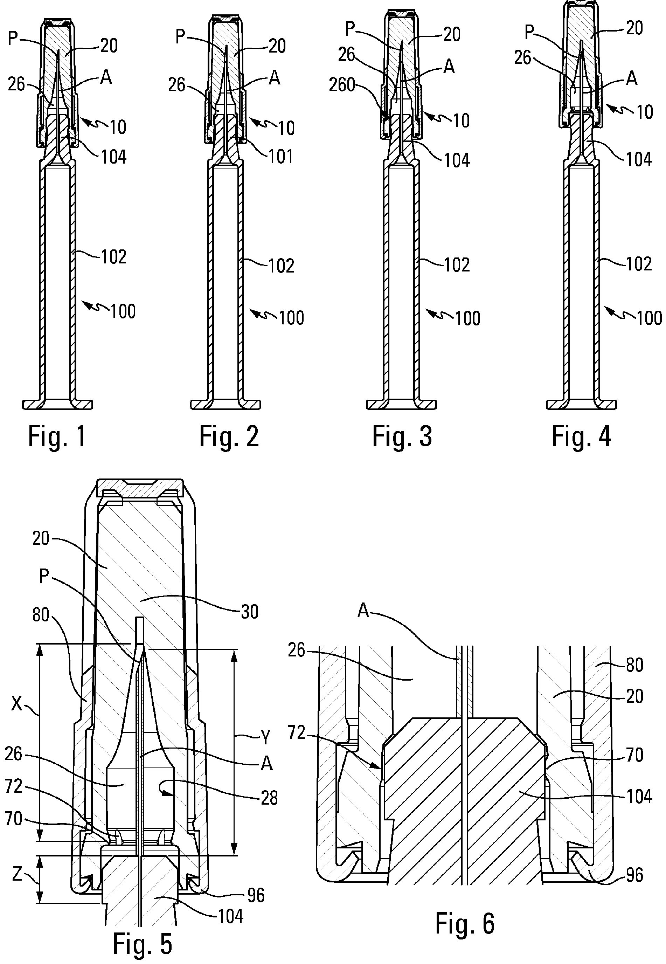

Un bourrelet annulaire 70 formant un renflement interne de matière est prévu dans la paroi latérale 28 du capuchon 20, comme visible notamment sur la figure 5. Ce bourrelet 70, comme notamment décrit dans le document EP1208861, est muni d'une pluralité de fentes 72, avantageusement quatre, s'étendant en direction longitudinale sur le bourrelet 70, ces fentes étant de préférence régulièrement angulairement réparties. On peut bien entendu prévoir un nombre quelconque de fentes 72, lesquelles peuvent présenter une profondeur plus ou moins importante. Si elles sont en grand nombre, ces fentes 72 séparent entre eux un grand nombre de portions du bourrelet 70 qui forment chacun une petite protubérance. De même, ces fentes 72 peuvent être plus ou moins larges. La longueur de chaque fente 72 est de préférence inférieure à la hauteur Z de la boule 104.

Comme illustré sur la figure 1, lorsque le protège-aiguille est en position de protection autour de l'aiguille A, l'extrémité libre, c'est-à-dire la pointe P, de l'aiguille A est plantée dans la paroi d'extrémité 30 du capuchon 20 tandis que la boule 104 de la seringue 100 pénètre au moins partiellement dans le logement 26 du capuchon 20, et coopère de manière étanche avec ledit bourrelet annulaire 70.

De manière connue notamment du document EP1208861, le bourrelet annulaire 70 coopère avec une paroi latérale de la boule 104 de la seringue 100, ce qui réalise la fermeture étanche de ladite cavité 26 par rapport à l'atmosphère. Au moment de l'insertion de la boule 104 du corps 102 de la seringue 100 dans la cavité 26 du capuchon 20, il y a un écrasement du bourrelet annulaire 70 par la boule 104 de la seringue, et les fentes 72 n'empêchent donc pas la fermeture étanche de la cavité 26.

Lorsque le protège-aiguille 10 est retiré, le volume de la cavité 26 va augmenter, comme visible sur les figures 1 et 2. Ladite cavité 26 étant obturé de manière étanche à la fois vis-à-vis de l'aiguille A et de la boule 104 de la seringue, il se crée donc une dépression dans ladite cavité 26.

Avec les protèges-aiguilles existants, la pointe de l'aiguille va être reliée à ladite cavité alors que celle-ci est encore en coopération étanche avec ladite boule de la seringue. La dépression dans la cavité va alors aspirer du liquide à travers ladite aiguille.

Selon l'invention, le protège-aiguille 10 est réalisé de telle sorte que pendant son retrait, la cavité 26 du capuchon 20 est nécessairement reliée à l'atmosphère avant que la pointe P de l'aiguille A ne soit reliée à ladite cavité 26.

Pour ce faire, le capuchon 20 du protège-aiguille 10 est de préférence réalisé conformément à l'équation suivante:

$$X < Y + Z/2$$

avec les définitions suivantes:

X = distance définie entre le point de fermeture étanche de la pointe P de l'aiguille A et le point d'étanchéité entre le capuchon 20 et la boule 104 de la seringue 100;
Y = longueur de l'aiguille entre la pointe P et la surface d'extrémité de la boule 104;
Z = hauteur de la boule 104.

Cette mise en oeuvre garantit que lorsque le protège-aiguille est retiré, les fentes 72 du bourrelet 70 vont toujours relier l'intérieur de la cavité 26 à l'atmosphère (comme visible sur la figure 6) avant que la pointe P de l'aiguille A ne sorte de son obturation étanche dans la paroi d'extrémité 30 du capuchon 20. De ce fait, au moment où ladite pointe P va être reliée avec ladite cavité 26, cette dernière ne sera plus sous dépression, et il n'y aura donc plus d'aspiration de liquide à travers ladite aiguille A.

Bien que la présente invention ait été décrite en référence à un mode de réalisation particulier, il est entendu que la présente invention n'est pas limitée par celui-ci mais qu'au contraire l'homme du métier peut y apporter toutes modifications utiles sans sortir du cadre de la présente invention tel que défini par les revendications annexées.

## Revendications

1. Ensemble de distribution comportant une seringue (100) comprenant un corps (102) pourvu d'une boule (104) supportant une aiguille (A) pourvue d'une pointe (P), et un dispositif de protection amovible (10) pour aiguille de seringue, ledit dispositif de protection (10) comportant un capuchon élastique (20) qui définit une cavité intérieure (26) délimitée par une paroi latérale (28) et par une paroi d'extrémité distale (30), ladite paroi d'extrémité (30) étant pleine et recevant de manière étanche, en position de protection, ladite pointe (P) de ladite aiguille (A) d'une seringue (100), ladite paroi latérale (28) comportant un bourrelet annulaire (70) pourvu d'au moins une fente (72) s'étendant en direction longitudinale sur ledit bourrelet annulaire (70), ledit bourrelet annulaire (70) coopérant de manière étanche, en position de protection, avec ladite boule (104) de la seringue, **caractérisé en ce que** ladite cavité (26) dudit capuchon (20) est dimensionnée de telle sorte que lorsque ledit dispositif de protection (10) est retiré de ladite seringue (100), ladite cavité (26) est reliée à l'atmosphère via ladite au moins une fente (72) avant que ladite pointe (P) de l'aiguille (A) soit reliée à ladite cavité (26).

2. Ensemble selon la revendication 1, dans lequel une pluralité, notamment quatre, fentes (72) régulièrement angulairement réparties s'étendent en direction longitudinale sur ledit bourrelet annulaire (70).

3. Ensemble selon la revendication 1 ou 2, dans lequel ladite au moins une fente (72) a une longueur inférieure à la hauteur (Z) de ladite boule (104).

4. Ensemble selon l'une quelconque des revendications précédentes, dans lequel ledit capuchon (20) est réalisé conformément à l'équation suivante:

$$X < Y + Z/2$$

avec X étant la distance entre le point de fermeture étanche de la pointe (P) de l'aiguille (A) et le point d'étanchéité entre le capuchon (20) et la boule (104), Y étant la longueur de l'aiguille (A) entre la pointe (P) et la surface d'extrémité de la boule (104), et Z étant la hauteur de la boule (104).

5. Ensemble selon l'une quelconque des revendications précédentes, comportant en outre une coque rigide (80) destinée à entourer en le contenant ledit capuchon (20) et étant pourvue de moyens de retenue (96) dudit capuchon (20).

## Patentansprüche

1. Ausgabeaufbau, umfassend eine Spritze (100), umfassend einen Körper (102), der mit einem Stöpsel (104) versehen ist, die eine Nadel (A) trägt, die mit einer Spitze (P) versehen ist, und eine abnehmbare Schutzvorrichtung (10) für eine Spritzennadel, wobei die Schutzvorrichtung (10) eine elastische Kappe (20) umfasst, die einen inneren Hohlraum (26) definiert, der durch eine Seitenwand (28) und durch eine distale Endwand (30) begrenzt ist, wobei die Endwand (30) massiv ist und in der Schutzposition die Spitze (P) der Nadel (A) einer Spritze (100) dichtend aufnimmt, wobei die Seitenwand (28) eine ringförmige Wulst (70) umfasst, die mit mindestens einem Schlitz (72) versehen ist, der sich in Längsrichtung auf der ringförmigen Wulst (70) erstreckt, wobei die ringförmige Wulst (70) in der Schutzposition dichtend mit dem Stöpsel (104) der Spritze zusammenwirkt, **dadurch gekennzeichnet, dass** der Hohlraum (26) der Kappe (20) derart bemessen ist, dass, wenn die Schutzvorrichtung (10) von der Spritze (100) abgezogen ist, der Hohlraum (26) über den mindestens einen Schlitz (72) mit der Atmosphäre verbunden ist, bevor die Spitze (P) der Nadel (A) mit dem Hohlraum (26) verbunden wird.

2. Aufbau nach Anspruch 1, wobei sich eine Vielzahl von insbesondere vier Schlitzen (72), die gleichmäßig winkelig verteilt sind, in Längsrichtung auf dem ringförmigen Wulst (70) erstreckt.

3. Aufbau nach Anspruch 1 oder 2, wobei der mindestens eine Schlitz (72) eine Länge aufweist, die kleiner ist als die Höhe (Z) des Stöpsels (104).

4. Aufbau nach einem der vorhergehenden Ansprüche, wobei die Kappe (20) entsprechend der folgenden Gleichung gestaltet ist:

$$X < Y + Z/2$$

wobei X der Abstand zwischen dem dichten Schließpunkt der Spitze (P) der Nadel (A) und dem Dichtungspunkt zwischen der Kappe (20) und dem Stöpsel (104) ist, Y die Länge der Nadel (A) zwischen der Spitze (P) und der Endfläche des Stöpsels (104) ist, und Z die Höhe des Stöpsels (104) ist.

5.  Aufbau nach einem der vorhergehenden Ansprüche, des Weiteren umfassend eine starre Schale (80), die dazu gedacht ist, die Kappe (20) zu umgeben und sie dabei aufzunehmen, und die mit Haltemitteln (96) der Kappe (20) versehen ist.

**Claims**

1.  A distribution assembly comprising both a syringe (100) having a body (102) provided with a tip (104) supporting a needle (A) having a bevel (P), and also a removable syringe needle protection device (10), said protection device (10) comprising a flexible cap (20) defining an inside cavity (26) defined by a side wall (28) and by a distal end wall (30), said end wall (30) being solid and, when in the protection position, receiving said bevel (P) of said needle (A) of a syringe (100) in airtight manner, said side wall (28) including an annular bead (70) provided with at least one slot (72) extending across said annular bead (70) in a longitudinal direction, said annular bead (70), when in the protection position, co-operating in airtight manner with said tip (104) of the syringe, the assembly being **characterized in that** said cavity (26) of said cap (20) is dimensioned in such a manner that when said protection device (10) is removed from said syringe (100), said cavity (26) is connected to the atmosphere via said at least one slot (72) before said bevel (P) of the needle (A) becomes connected to said cavity (26).

2.  An assembly according to claim 1, wherein a plurality, and in particular four, slots (72) that are regularly distributed angularly extend in the longitudinal direction across said annular bead (70).

3.  An assembly according to claim 1 or claim 2, wherein said at least one slot (72) is of a length shorter than the height (Z) of said tip (104).

4.  An assembly according to any preceding claim, wherein said cap (20) is made in compliance with the following equation:

$$X < Y + Z/2$$

where X is the distance between the point at which the bevel (P) of the needle (A) is closed in airtight manner and the point at which the cap (20) and the tip (104) are connected together in airtight manner, Y being the length of the needle (A) between the bevel (P) and the end surface of the tip (104), and Z being the height of the tip (104).

5.  An assembly according to any preceding claim, further including a rigid shell (80) for surrounding and containing said cap (20) and provided with retention means (96) for retaining said cap (20).

Fig. 1

Fig. 2

Fig. 3

Fig. 4

Fig. 5

Fig. 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0429052 A
- EP 0976415 A
- EP 1208861 A
- WO 2015052417 A